# EUROPÄISCHE PATENTSCHRIFT

(11) **EP 2 594 453 B1**
(45) Veröffentlichungstag und Bekanntmachung des Hinweises auf die Patenterteilung: **30.05.2018**
(21) Anmeldenummer: 12192290.0
(22) Anmeldetag: 12.11.2012
(51) Int. Cl.: B62B 3/00, A61G 12/00

(54) **Transportwagen, insbesondere für Sterilgut**
Trolley, particularly for sterile goods
Chariot de transport, notamment pour produits stériles

(30) Priorität: 16.11.2011 DE 102011055413
(43) Veröffentlichungstag der Anmeldung: 22.05.2013
(73) Patentinhaber: HUPFER Metallwerke GmbH & Co. KG, 48653 Coesfeld (DE)
(72) Erfinder: Schumacher, Helmut, 48653 Coesfeld (DE); Flück, Egbert, 46348 Raesfeld (DE); Eggemann, Carsten, 48249 Dülmen (DE)
(74) Vertreter: Lorenz, Bernd Ingo Thaddeus

(56) Entgegenhaltungen:
- DE-A1- 3 619 262
- US-A- 4 846 537
- US-A- 5 797 503

## Beschreibung

Die Erfindung betrifft einen Transportwagen, insbesondere für Sterilgut, mit einem Fahrgestell, einem auf dem Fahrgestell angeordneten Gehäuse, das eine Rückwand sowie zwei Seitenwände aufweist und an seiner Frontseite durch eine ein- oder zweiflügelige Tür verschlossen ist, und mit mindestens einer höhenverstellbaren Auflage. Die Auflage ist auf Verbindern abgestützt, die sich parallel zu den Seitenwänden des Gehäuses erstrecken und an Vertikalschienen im Innenraum des Gehäuses befestigt sind. Der Transportwagen kann in reinen und unreinen Klinikbereichen zum sicheren und damit hygienisch einwandfreien Transport von Sterilgut verwendet werden. Die Auflage besteht häufig aus einem Drahtrost oder einem Lochblech. Unterschiedliche Ausgestaltungen der Auflage lassen den Transport und die Lagerung von Sterilgut in Körben, Siebschalen, Containern, Sterilfolienverpackungen und Einwegverpackungen zu.

Bei einem aus der Praxis bekannten Transportwagen, von dem die Erfindung ausgeht, bestehen die Vertikalschienen aus Holmen, an denen vorstehende Stifte angeordnet sind. Die Verbinder zur Abstützung eines Rostes oder einer sonstigen Auflage weisen an ihren Enden abgewinkelte Laschen auf, wobei eine Lasche eine Bohrung und die andere Lasche eine C-förmige Ausstanzung enthält. Die Verbinder werden an ihrem einen Ende auf einen vorstehenden Zapfen der Holme aufgesteckt und an ihrem anderen Ende an einem vorstehenden Zapfen angehängt. Die Stabilität der Aufhängung ist noch verbesserungsbedürftig. Ferner besteht die Gefahr, dass Papier- oder Folienverpackungen des Sterilgutes an den vorstehenden Zapfen der Holme beschädigt werden.

Die DE 101 56 602 C1 offenbart einen Transportwagen insbesondere für Sterilgut, mit vertikalen Stützträgern. Die Stützträger sind mit vorzugsweise äquidistant angeordneten Ausschnitten versehen, in welche Tragbügel einhängbar sind, an denen Transportbehälter angeordnet werden können. Die Ausschnitte sind gemäß der Zeichnungen sowohl an den vorderen als auch an den hinteren vertikalen Stützträgern frontseitig angeordnet.

Ein weiterer Transportwagen, insbesondere für Sterilgut, ist in der DE 101 55 515 A1 beschrieben. Dabei sind alle Bestandteile des Rahmens, einschließlich vertikal verlaufender Stützträger, als wenigstens einseitig offene Profile ausgeführt. Zur direkten Aufnahme von Einlegeelementen sind dabei Aussparungen in den Stützträgern vorgesehen, welche bevorzugt an den innenliegenden Schenkeln der Profil angeordnet sind.

Die Patentschrift US 3,199,683 offenbart einen Transportwagen mit vertikalen Holmen. An jeweils zwei parallelen Innenflächen benachbarter Holme sind Schlitze zur Aufnahme von Gleitschienen vorgesehen, auf denen Bleche, Pfannen und dergleichen abgestützt werden können.

Die Offenlegungsschrift US 5,797,503 A zeigt einen offenen Transportwagen gemäß dem Oberbegriff des geltenden Patentanspruches 1. Einen ähnlichen Offenbarungsgehalt hat auch DE 36 19 262 A1.

Der Erfindung liegt die Aufgabe zugrunde, einen Transportwagen für Sterilgut anzugeben, dessen Auflagen für das Sterilgut ohne Werkzeug in einem vorgegebenen Raster höhenverstellbar sind. Die Position, Anzahl sowie auch die Art der Auflagen sollen jederzeit veränderbar und austauschbar sein. Dabei sind sowohl an den Vertikalschienen als auch an den Verbindern zur Abstützung der Auflagen vorstehende Teile an denen Folien- oder Papierverpackungen des Sterilgutes beschädigt werden können, zu vermeiden.

Gegenstand der Erfindung und Lösung dieser Aufgabe ist ein Transportwagen nach Anspruch 1.

Erfindungsgemäß sind die Verbinder als Einhängeverbinder ausgebildet, die an ihrem einen Ende in Schlitze einer an der Frontseite des Gehäuses angeordneten Vertikalschiene und an ihrem anderen Ende in Schlitze einer zur Gehäuserückwand benachbarten Vertikalschiene eingehängt werden können. Erfindungsgemäß sind die Schlitze der frontseitigen Vertikalschiene und die Schlitze der zur Gehäuserückwand benachbarten Vertikalschiene in Einhängeflächen der Vertikalschienen angeordnet, die zueinander orthogonal ausgerichtet sind. Die Einhängefläche einer Vertikalschiene erstreckt sich somit parallel zur Rückwand, während die Einhängefläche der anderen Vertikalschiene parallel zur Seitenwand des Gehäuses ausgerichtet ist. Die Schlitze der Vertikalschienen erstrecken sich insbesondere vertikal und sind vorzugsweise mit einem zwischen 50 mm und 70 mm gewählten einheitlichen Rastermaß in den Vertikalschienen angeordnet. Besonders bevorzugt ist ein Rastermaß von etwa 60 mm.

Gemäß der Erfindung erstreckt sich die Einhängefläche der frontseitigen Vertikalschiene parallel zur Seitenwand des Gehäuses. Die Seitenwände des Gehäuses weisen an der Frontseite ein im Querschnitt U-förmiges Zargenprofil auf, an welches die Einhängefläche einstückig angeformt sein kann. Die Schlitze in der zur Seitenwand parallelen Einhängefläche der frontseitigen Vertikalschiene legen die korrekte Lage des Verbinders fest und sichern den Verbinder gegen axiales Verschieben.

An den Abstand, der bei der Fertigung des Gehäuses zwischen der Einhängefläche der frontseitigen Vertikalschiene und den an der Rückwand angeordneten Vertikalschienen eingehalten werden muss, werden keine besonderen Toleranzanforderungen gestellt. Die Einhängeverbinder sind in den Schlitzen der rückseitigen Vertikalschiene schwimmend gelagert. Die in Schlitzen der rückseitigen Vertikalschienen eingeführten Steckelemente geben den Einhängeverbindern seitlichen Halt und nehmen Querkräfte auf.

Die Einhängefläche der zur Rückwand des Gehäuses benachbarten Vertikalschiene ist erfindungsgemäß mit Abstand vor der Rückwand angeordnet, wobei der Abstand der Rückwand so bemessen ist, dass die den Schlitz durchfassenden Teile des Einhängeverbinders ausreichend weit überstehen können. Die rückseitige Vertikalschiene kann dabei aus einem Winkelprofil bestehen, welches an der Rückwand, einer Seitenwand oder einer die Gehäuseecke überbrückende Leiste befestigt ist.

Vorzugsweise sind die Einhängeverbinder als Stanz- und Biegeteile aus Blech gefertigt und weisen an ihren Enden Anschlussfahnen auf, die in die Schlitze der Vertikalschienen einsetzbar sind. Die Montage ist ohne Werkzeug möglich. Die Einhängeverbinder weisen an einem Ende eine L-förmig abgewinkelte und mit einem Einsteckschlitz versehene Anschlussfahne auf, die auf die untere Berandung der Schlitze in der zugeordneten Vertikalschiene aufsteckbar ist. Die Anschlussfahne ist in die Schlitze der Einhängefläche einführbar, die sich parallel zur Seitenwand des Gehäuses erstreckt. Üblicherweise handelt es sich um die frontseitige Vertikalschiene. An ihrem anderen Ende weisen die Einhängeverbinder eine Anschlussfahne auf, die sich parallel zur Seitenwand des Gehäuses erstreckt und in die Schlitze der rückseitigen Vertikalschiene einführbar sind, deren Einhängefläche sich parallel zur Rückwand des Gehäuses erstreckt. Die Anschlussfahne des Einhängeverbinders weist eine an die Länge der Schlitze angepasste Breite auf. Die in einen Schlitz der rückseitigen Einhängeschiene eingeführte Anschlussfahne nimmt auf den Einhängeverbinder wirkende Querkräfte und Biegemomente auf und sichert den Einhängeverbinder vertikal.

Zur Aufnahme großer Querkräfte und Kippmomente eignet sich eine Ausführung des Einhängeverbinders mit zwei Anschlussfahnen, die sich parallel zur Seitenwand erstrecken und in zueinander beabstandete Schlitze der zur Rückwand des Gehäuses benachbarten Vertikalschienen einsteckbar sind. Während eine der beiden Anschlussfahnen eine an die Länge der Schlitze angepasste Breite aufweist, ist die andere Anschlussfahne zweckmäßig deutlich schmaler ausgebildet.

Bei allen zuvor beschriebenen Ausführungen ist die Blechstärke der Anschlussfahnen vorzugsweise auf die Spaltbreite der in den Vertikalschienen vorgesehenen Schlitze so abgestimmt, dass die Anschlussfahnen in den Schlitzen mit einem seitlichen Spiel von weniger als 0,5 mm angeordnet sind. Bevorzugt ist ein Spiel von 0,1 bis 0,3 mm. Die Montage ist trotz der engen Toleranzen problemlos. Zunächst wird der Einhängeverbinder in mindestens einen Schlitz einer an der Rückwand des Gehäuses angeordneten Vertikalschiene eingeschoben. Danach kann die Einhängeschiene mit einer seitwärts gerichteten Bewegung in einen Schlitz der frontseitigen Vertikalschiene eingehängt werden. Die Abmessungen und Materialstärke der Anschlussfahnen und die Geometrie der Schlitze können mit engen Toleranzen aufeinander abgestimmt werden, ohne dass der Montagevorgang hierdurch erschwert wird. Durch enge Toleranzen, die zwischen den Anschlussfahnen und der Geometrie der Schlitze eingehalten werden, ist der Einhängeverbinder fest und mit strammen Sitz an den Vertikalschienen befestigbar.

Die Geometrie der Einhängeverbinder und ihre Stützflächen sind auf die Auflage abzustimmen. Die Einhängeverbinder können insbesondere einen in Richtung auf die Seitenwand zurückspringenden mittleren Halteabschnitt mit einer Stützfläche für die Auflage und dazu parallel versetzt angeordnete Endabschnitte aufweisen. Eine in den Halteabschnitt eingehängte Auflage, z. B. in Form eines Drahtrostes, eines Lochbleches, eines Korbes oder dergleichen, ist durch die in den Gehäuseinnenraum vorspringenden Endabschnitte des Einhängeverbinders lagegesichert.

Zur Verbesserung der Formstabilität können die Einhängeverbinder an ihrem unteren Rand eine Abkantung aufweisen.

Die an den Einhängeverbindern abgestützte Auflage kann vielfältig ausgestaltet sein. Die Auflage kann insbesondere als Drahtrost, als ebene Fläche, Schale oder als Korb ausgebildet sein, wobei die Auflage je nach Ausführung an den Einhängeverbindern aufgehängt ist oder auf vorspringenden Flächen der Einhängeverbinder aufliegen kann.

Der Transportwagen kann erfindungsgemäß auch mit einer oder mehreren herausziehbaren Auflagen ausgestattet werden, die wie alle anderen Auflagen in dem vorgegebenen Höhenraster der Vertikalschienen variabel und ohne Werkzeug verstellt werden können. Für eine herausziehbare Auflage werden Einhängeverbinder verwendet, an denen jeweils eine Gleitschiene befestigt ist. Die Auflage weist Seitenteile auf, die an den Gleitschienen zwischen Anschlagelementen verschiebbar geführt sind. Vorzugsweise bestehen die Gleitschienen aus Kunststoffleisten und sind an die Seitenteile der Auflage Profile angeschlossen oder durch Blechumformung einteilig angeformt, welche die Gleitschienen U-förmig umgreifen. An der Unterseite der Gleitschienen können Zapfen befestigt sein, welche den Schiebeweg der Auflage begrenzen. Um Sterilgüter auf den Auflagen abzustellen oder von der Auflage zu entnehmen, kann die Auflage um bis zu einem Drittel aus dem Gehäuse des Transportwagens herausgezogen werden. Bei einer standardmäßigen Gehäusetiefe von 600 mm ragt die Auflage somit ca. 200 mm nach vorne aus dem Wagen. Dies erleichtert die Handhabung beachtlich.

Die Auflage weist zweckmäßig einen aus Seitenteilen und Querträgern gebildeten Rahmen auf. Die Auflagefläche kann aus einem Drahtrost, einem Lochblech oder dergleichen bestehen.

Der erfindungsgemäße Transportwagen kann als geschlossener Sterilguttransportwagen eingesetzt werden, dessen Gehäuse aus einem stabilen, selbsttragenden Korpus aus Edelstahl gefertigt ist. An seiner Frontseite weist der Wagen eine ein- oder zweiflügelige Tür auf, die mit einem Schwenkwinkel von bis zu 270° schwenkbar sind. Mit einem vorgegebenen Rastermaß, welches durch die Anordnung der Schlitze der Vertikalschienen vorgegeben ist, können Auflagen eingesetzt werden, wobei Drahtrostauflagen, geschlossene Auflagen und sonstige Auflagen aus einem Standardregalprogramm verwendet werden können. Die Auflagen lassen sich einfach und schnell in die Einhängeverbinder einhängen und wieder entnehmen. Der Innenraum des Transportwagens ist glatt und lässt sich dadurch gut reinigen. Durch Versetzen der Einhängeverbinder können die Auflagen in einem vorgegebenen Raster, welches durch die Anordnung der Schlitze der Vertikalschienen festgelegt ist, variabel versetzt werden. Dadurch sind sowohl die Position, Anzahl als auch die Art der Auflagen jederzeit veränderbar und austauschbar. Insbesondere kann der Transportwagen auch mit einem oder mehreren herausziehbaren Auflagen ausgestattet werden.

Im Folgenden wird die Erfindung anhand von Ausführungsbeispielen erläutert. Es zeigen schematisch:
- **Fig. 1**: einen geöffneten Transportwagen für Sterilgut mit zwei höhenverstellbaren Auflagen,
- **Fig. 2**: im Ausschnitt eine Ansicht des Innenraums des in Fig. 1 dargestellten Transportwagens,
- **Fig. 3**: die Befestigung eines Einhängeverbinders an der Frontseite des in Fig. 1 dargestellten Transportwagens,
- **Fig. 4**: die Befestigung eines Einhängeverbinders an der Rückwand des in Fig. 1 dargestellten Transportwagens,
- **Fig. 5**: eine unterseitige Ansicht eines Einhängeverbinders für eine verschiebbare Auflage,
- **Fig. 6**: die Befestigung des in Fig. 5 dargestellten Einhängeverbinders an der Rückwand des Transportwagens,
- **Fig. 7**: eine Einzelteilzeichnung des in den Fig. 5 und 6 dargestellten Einhängeverbinders.

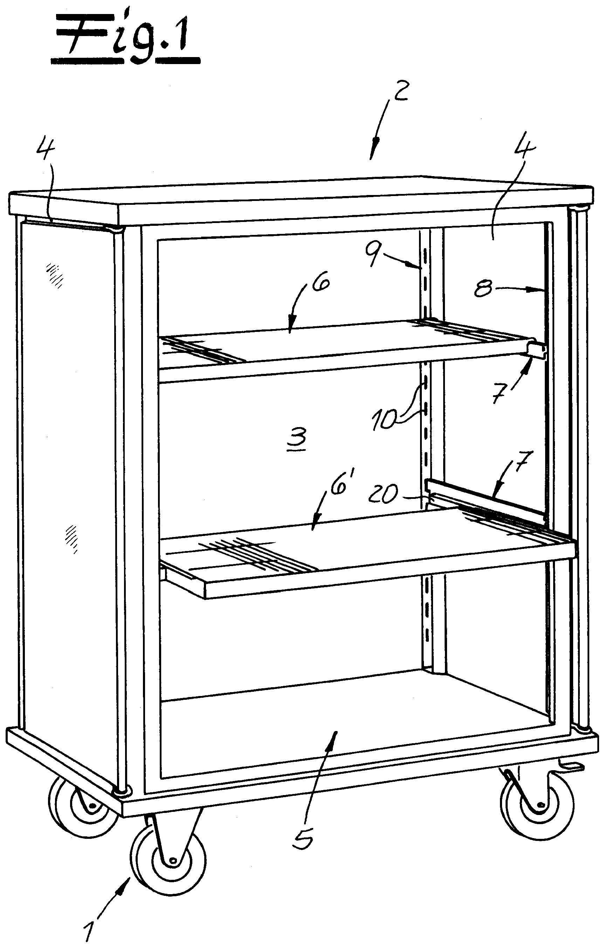

In Fig. 1 ist ein Transportwagen für Sterilgut dargestellt, der in reinen und unreinen Klinikbereichen zum sicheren und hygienisch einwandfreien Transport von Sterilgut verwendet werden kann, und zwar sowohl bei der Versorgung als auch bei der Entsorgung. Der Transportwagen besteht in seinem grundsätzlichen Aufbau aus einem Fahrgestell 1 und einem auf dem Fahrgestell angeordneten kastenförmigem Gehäuse 2, das eine Rückwand 3 sowie zwei Seitenwände 4 aufweist und an seiner Frontseite 5 durch eine ein- oder zweiflügelige Tür verschlossen ist. Die Fig. 1 zeigt den geöffneten Transportwagen. Der Transportwagen ist mit einer oder mehreren höhenverstellbaren Auflagen 6, 6' ausgestattet. Die Auflagen 6, 6' sind auf Verbindern 7 abgestützt, die an Vertikalschienen 8, 9 im Innenraum des Gehäuses 2 befestigt sind und sich parallel zu den Seitenwänden 4 des Gehäuses 2 erstrecken.

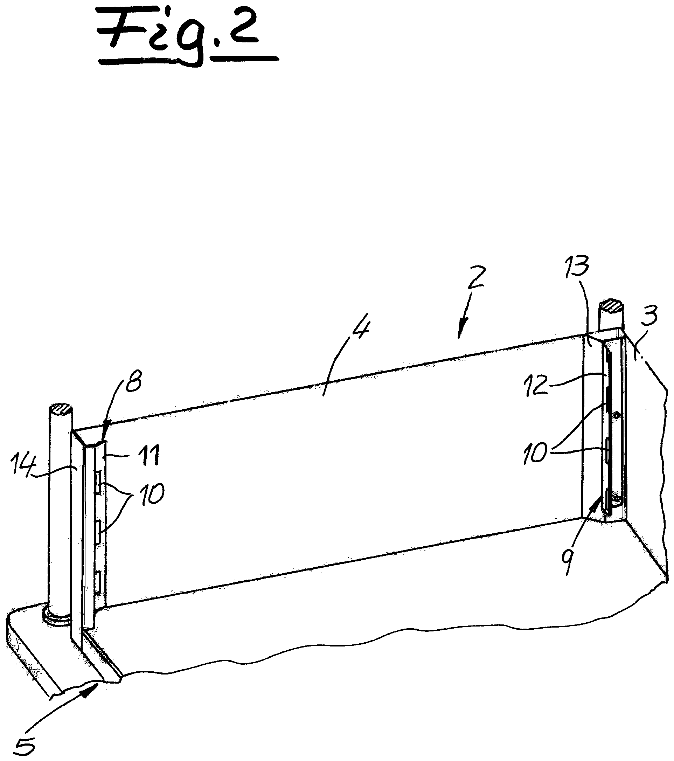

Die Fig. 2 zeigt im Ausschnitt eine innenseitige Ansicht des Gehäuses 2, und zwar in der Blickrichtung auf die linke Seitenwand 4 des Transportwagens. Die Vertikalschienen 8, 9 enthalten vertikal ausgerichtete Schlitze 10, die in einem zwischen 50 mm und 70 mm gewählten einheitlichen Rastermaß angeordnet sind. Bevorzugt ist ein Rastermaß von ca. 60 mm. Die Schlitze 10 der frontseitigen Vertikalschiene 8 und die Schlitze der zur Gehäuserückwand benachbarten Vertikalschiene 9 sind in Einhängeflächen 11, 12 angeordnet, die zueinander orthogonal ausgerichtet sind. Gemäß der Darstellung in Fig. 2 erstreckt sich die Einhängefläche 11 der frontseitigen Vertikalschiene 8 parallel zur Seitenwand 4 des Gehäuses 2 während die Einhängefläche 12 der zur Rückwand 3 des Gehäuses 2 benachbarten Vertikalschiene 9 mit Abstand vor der Rückwand 3 angeordnet und parallel zur Rückwand 3 ausgerichtet ist. Die rückseitige Vertikalschiene 9 besteht aus einem Winkelprofil, welches an einer die Gehäuseecke überbrückenden Leiste 13 befestigt ist. Alternativ kann das Winkelprofil auch an der Rückwand 3 oder an der Seitenwand 4 befestigt sein. Die Seitenwand 4 des Gehäuses 2 weist an der Frontseite 5 ein im Querschnitt U-förmiges Zargenprofil 14 auf. Der freie Schenkel des Zargenprofils 14 kann gleichzeitig als frontseitige Vertikalschiene genutzt werden. Die geschlitzte Einhängefläche 11 ist an das Zargenprofil 14 einstückig angeformt. Alternativ besteht gemäß der Erfindung auch die Möglichkeit, die Vertikalschiene 8 als Winkelprofil auszubilden und an dem Zargenprofil 14 zu befestigen.

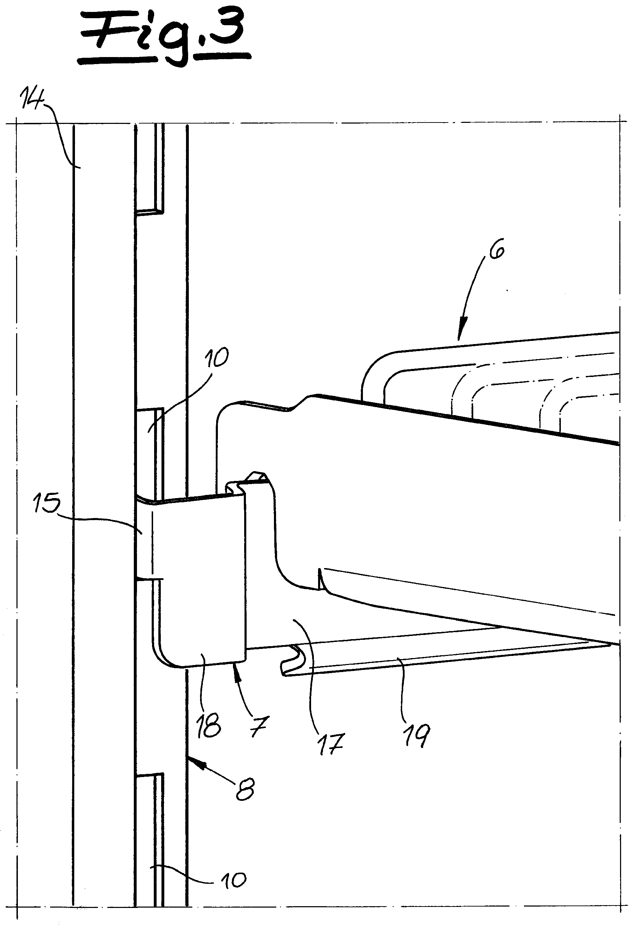

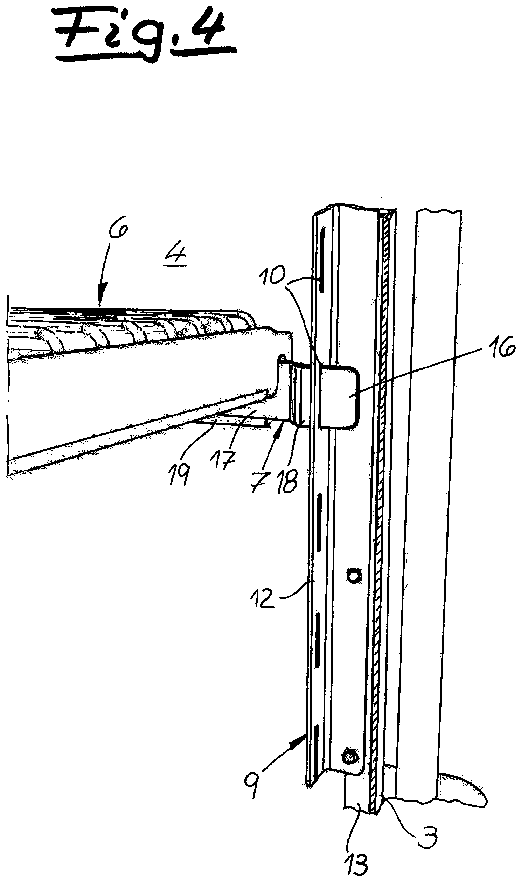

Die Verbinder 7 zur Abstützung der Auflage 6 sind als Einhängeverbinder ausgebildet, die ohne Werkzeug in die Vertikalschienen 8, 9 eingehängt werden können. In den Fig. 3 und 4 ist die Befestigung eines Einhängeverbinders an der frontseitigen Vertikalschiene 8 und an der zur Rückwand benachbarten Vertikalschiene 9 dargestellt. Der Einhängeverbinder 7 ist als Stanz- und Biegeteil aus Blech gefertigt und weist an seinen Enden Anschlussfahnen 15, 16 auf, die in die Schlitze 10 der Vertikalschienen 8, 9 einsetzbar sind. Die Fig. 3 zeigt die Befestigung des Einhängeverbinders 7 an der frontseitigen Vertikalschiene 8. An dem zugeordneten Ende weist der Einhängeverbinder 7 eine L-förmig abgewinkelte und mit einem Einsteckschlitz versehene Anschlussfahne 15 auf, die auf die untere Berandung des Schlitzes 10 der frontseitigen Vertikalschiene aufsteckbar ist. An ihrem anderen Ende weist der Einhängeverbinder 7 eine Anschlussfahne 16 auf, die sich parallel zur Seitenwand 4 des Gehäuses 2 erstreckt und eine an die Länge der Schlitze 10 angepasste Breite aufweist (Fig. 4). Die Blechstärke der Anschlussfahnen 15, 16 ist auf die Spaltbreite der in den Vertikalschienen 8, 9 vorgesehenen Schlitze 10 so abgestimmt, dass die Anschlussfahnen 15, 16 in den Schlitzen 10 mit einem seitlichen Spiel von weniger als 0,5 mm, vorzugsweise mit einem Spiel zwischen 0,1 mm und 0,3 mm, angeordnet sind. Dadurch ist ein fester Sitz des Einhängeverbinders 7 an den Vertikalschienen 8, 9 gewährleistet.

Den Darstellungen in den Fig. 3 und 4 ist auch zu entnehmen, dass der Einhängeverbinder 7 einen in Richtung auf die Seitenwand 4 zurückspringenden mittleren Halteabschnitt 17 mit einer Stützfläche für die Auflage 6 und zu dem Halteabschnitt 17 parallel versetzt angeordnete Endabschnitte 18 aufweist. Die Auflage 6, z. B. eine Drahtrostauflage, kann in den Einhängeverbinder 7 eingehängt werden. Zur Verbesserung der Formstabilität weist der Einhängeverbinder 7 ferner an seinem unteren Rand eine Abkantung 19 auf. Im Ausführungsbeispiel besteht die Auflage 6 aus einem Drahtrost. Die Auflage kann auch als geschlossene Auflage mit einer ebenen Fläche, als Schale oder als Korb ausgebildet sein.

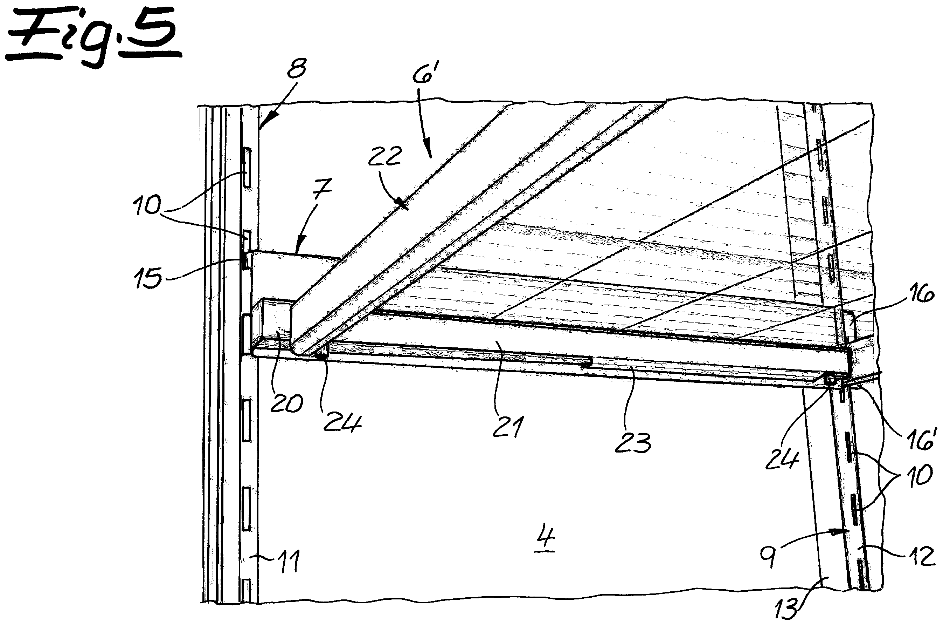

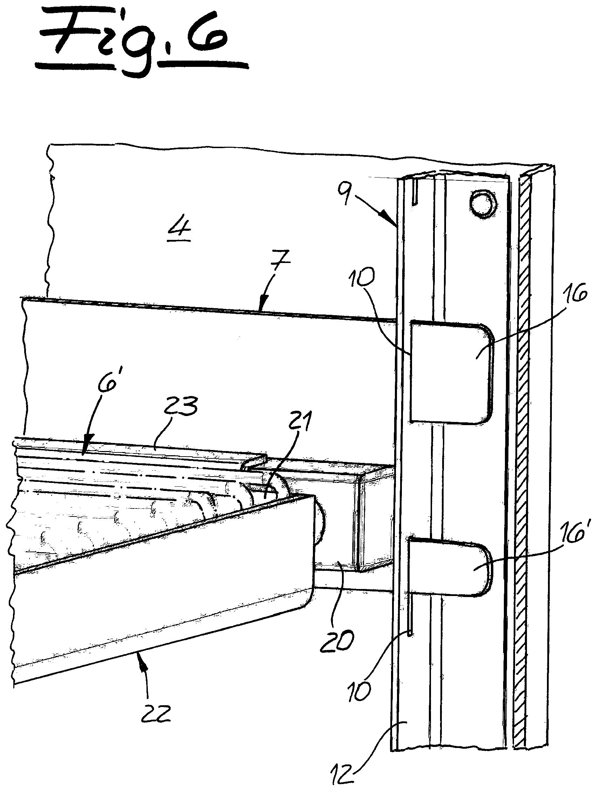

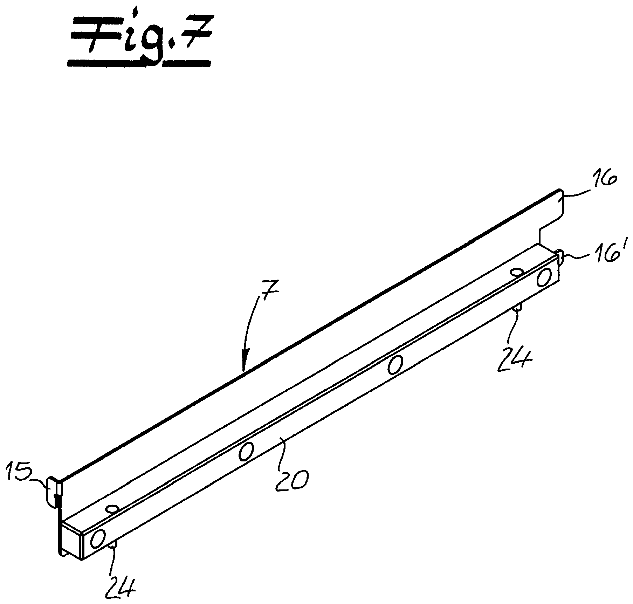

Der in Fig. 1 dargestellte Transportwagen ist beispielsweise mit einer fest eingehängten Drahtrostauflage 6 und einer ausziehbaren Drahtrostauflage 6' ausgestattet. Die ausziehbare Drahtrostauflage 6' kann um ein Drittel ausgezogen werden. Bei einer Tiefe von ca. 600 mm ragt die Auflage somit ca. 200 mm vorne aus dem Wagen. Ein Einhängeverbinder 7 für eine ausziehbare Auflage 6' ist in den Fig. 5 bis 7 dargestellt. Der Einhängeverbinder 7 ist als Stanz- und Biegeteil aus Blech gefertigt und weist an seinen Enden Anschlussfahnen 15, 16 auf, die in die Schlitze 10 der Vertikalschiene 8, 9 einsetzbar sind. An dem Einhängeverbinder 7 ist eine Gleitschiene 20 befestigt, die vorzugsweise aus einer Kunststoffleiste, z. B. aus Polyethylen, besteht. Die Auflage 6' weist einen aus Seitenteilen 21 und Querträgern gebildeten Rahmen 22 auf. An den Seitenteilen 21 sind Profile 23 angeordnet, welche die Gleitschienen 20 U-förmig umgreifen, so dass das Seitenteil 21 als Schlitten an der Gleitschiene 20 geführt ist. Das Profil 23 kann einteilig an dem Seitenteil 21 angeformt sein und aus einem durch Blechumformung gebildeten Abschnitt des Seitenteils bestehen. An der Unterseite der Gleitschiene 20 sind Zapfen 24 befestigt, welche den Schiebeweg der Auflage 6' begrenzen.

Der Einhängeverbinder 7 weist an seinem vorderen Ende eine L-förmig abgewinkelte und mit einem Einsteckschlitz versehene Anschlussfahne 15 auf, die in Schlitze 10 der frontseitigen Vertikalschiene 9 eingehängt werden kann. An seinem anderen Ende weist der Einhängeverbinder 7 zwei Anschlussfahnen 16, 16' auf, die sich parallel zur Seitenwand 4 erstrecken und in zueinander beabstandete Schlitze 10 der Vertikalschiene 8 einsteckbar sind. Eine der beiden Anschlussfahnen 16 weist eine an die Länge der Schlitze 10 angepasste Breite auf, während die andere Anschlussfahne 16' deutlich schmaler ausgebildet ist. Der Einhängeverbinder 7 ist in der Lage, große Querkräfte und Kippmomente, die bei einer Beladung der ausgezogenen Auflage 6' auftreten können, aufzunehmen.

## Patentansprüche

1. Transportwagen, insbesondere für Sterilgut, mit einem Fahrgestell (1) und mindestens einer höhenverstellbaren Auflage (6), wobei die Auflage (6) auf Einhängeverbindern (7) abgestützt ist, die an Vertikalschienen (8, 9) befestigt sind, **dadurch gekennzeichnet,**
**dass** auf dem Fahrgestell (1) ein Gehäuse (2) angeordnet ist, das eine Rückwand (3) sowie zwei Seitenwände (4) aufweist und an seiner Frontseite (5) durch eine ein- oder zweiflügelige Tür verschlossen ist, wobei die Vertikalschienen (8, 9) im Innenraum des Gehäuses befestigt sind und sich parallel zu den Seitenwänden (4) des Gehäuses (2) wobei die Einhängeverbinder(7) an ihrem einen Ende in Schlitze (10) einer an der Frontseite (5) des Gehäuses angeordneten Vertikalschiene (8) und an ihrem anderen Ende in Schlitze (10) einer zur Gehäuserückwand (4) benachbarten Vertikalschiene (9) eingehängt werden können, wobei die Schlitze (10) der frontseitigen Vertikalschiene (8) und die Schlitze (10) der zur Gehäuserückwand benachbarten Vertikalschiene (9) in Einhängeflächen (11, 12) der Vertikalschienen (8, 9) angeordnet sind, die zueinander orthogonal ausgerichtet sind, dass die Seitenwände (4) des Gehäuses (2) an der Frontseite (5) ein im Querschnitt U-förmiges Zargenprofil (14) aufweisen,
**dass** die Einhängefläche (11) der frontseitigen Vertikalschienen (8) sich parallel zur Seitenwand (4) des Gehäuses (2) erstreckt und an das Zargenprofil (14) einstückig angeformt ist oder die frontseitige Vertikalschiene (8) als Winkelprofil ausgebildet und an dem Zargenprofil (14) befestigt ist und
**dass** die Einhängefläche (11) der zur Rückwand (4) des Gehäuses (2) benachbarten rückseitigen Vertikalschienen (9) mit Abstand vor der Rückwand (4) angeordnet ist.

2. Transportwagen nach Anspruch 1, **dadurch gekennzeichnet, dass** die Schlitze (10) der Vertikalschienen (8, 9) vertikal ausgerichtet sind.

3. Transportwagen nach Anspruch 1 oder 2, **dadurch gekennzeichnet, dass** die rückseitige Vertikalschiene (9) aus einem Winkelprofil besteht, welches an der Seitenwand (4) oder einer die Gehäuseecke überbrückenden Leiste (13) befestigt ist.

4. Transportwagen nach einem der Ansprüche 1 bis 6, **dadurch gekennzeichnet, dass** die Einhängeverbinder (7) als Stanz- und Biegeteile aus Blech gefertigt sind und an ihren Enden Anschlussfahnen (15, 16) aufweisen, die in die Schlitze (10) der Vertikalschienen (8, 9) ohne Werkzeug einsetzbar sind.

5. Transportwagen nach Anspruch 4, **dadurch gekennzeichnet, dass** die Einhängeverbinder (7) an einem Ende eine L-förmig abgewinkelte und mit einem Einsteckschlitz versehene Anschlussfahne (15) aufweisen, die auf die untere Berandung der Schlitze (10) in den Vertikalschienen (8) aufsteckbar ist.

6. Transportwagen nach Anspruch 5, **dadurch gekennzeichnet, dass** die Einhängeverbinder (7) an ihrem anderen Ende eine Anschlussfahne (16) aufweisen, die sich parallel zur Seitenwand (4) des Gehäuses (2) erstreckt und eine an die Länge der Schlitze (10) angepasste Breite aufweist.

7. Transportwagen nach Anspruch 5, **dadurch gekennzeichnet, dass** die Einhängeverbinder (7) an ihrem anderen Ende zwei Anschlussfahnen (16, 16') aufweisen, die sich parallel zur Seitenwand (4) erstrecken und in zueinander beabstandete Schlitze (10) der Vertikalschiene (9) einsteckbar sind.

8. Transportwagen nach Anspruch 5, **dadurch gekennzeichnet, dass** eine der beiden Anschlussfahnen (16) eine an die Länge der Schlitze (10) angepasste Breite aufweist und die andere Anschlussfahne (16') schmaler ausgebildet ist.

9. Transportwagen nach einem der Ansprüche 4 bis 8, **dadurch gekennzeichnet, dass** die Blechstärke der Anschlussfahnen (15, 16) auf die Spaltbreite der in den Vertikalschienen (8, 9) vorgesehenen Schlitze (10) so abgestimmt ist, dass die Anschlussfahnen (15, 16) in den Schlitzen (10) mit einem seitlichen Spiel von weniger als 0,5 mm angeordnet sind.

10. Transportwagen nach einem der Ansprüche 1 bis 9, **dadurch gekennzeichnet, dass** die Schlitze (10) mit einem zwischen 50 mm und 70 mm gewählten einheitlichen Rastermaß in den Vertikalschienen (8, 9) angeordnet sind.

11. Transportwagen nach einem der Ansprüche 1 bis 10, **dadurch gekennzeichnet, dass** die Einhängeverbinder (7) einen in Richtung auf die Seitenwand (4) zurückspringenden mittleren Halteabschnitt (17) mit einer Stützfläche für die Auflage (6) und zu dem Halteabschnitt parallel versetzt angeordnete Endabschnitte (18) aufweisen.

12. Transportwagen nach einem der Ansprüche 1 bis 11, **dadurch gekennzeichnet, dass** die Einhängeverbinder (7) an ihrem unteren Rand eine Abkantung (19) zur Verbesserung der Formstabilität aufweisen.

13. Transportwagen nach einem der Ansprüche 1 bis 12, **dadurch gekennzeichnet, dass** die Auflage (6) als Drahtrost, ebene Fläche, Schale oder als Korb ausgebildet ist und an den Einhängeverbindern (7) aufgehängt ist oder auf vorspringenden Flächen der Einhängeverbinder aufliegt.

14. Transportwagen nach einem der Ansprüche 1 bis 13, **dadurch gekennzeichnet, dass** an den Einhängeverbindern (7) eine Gleitschiene (20) befestigt ist und dass die Auflage (6') Seitenteile (21) aufweist, die an den Gleitschienen (20) zwischen Anschlagelementen verschiebbar geführt sind.

15. Transportwagen nach Anspruch 14, **dadurch gekennzeichnet, dass** die Gleitschienen (20) aus Kunststoffleisten bestehen und dass an die Seitenteile (21) Profile (23) angeschlossen oder angeformt sind, welche die Gleitschienen (20) U-förmig umgreifen.

16. Transportwagen nach Anspruch 15, **dadurch gekennzeichnet, dass** an der Unterseite der Gleitschienen (20) Zapfen (24) befestigt sind, welche den Schiebeweg der Auflage (6') begrenzen.

17. Transportwagen nach einem der Ansprüche 14 bis 16, **dadurch gekennzeichnet, dass** die Auflage (6') einen aus den Seitenteilen (21) und Querträgern gebildeten Rahmen (22) aufweist.

## Claims

1. A trolley, in particular for sterile materials, with a chassis (1) and at least one height-adjustable support (6) wherein the support (6) is supported by hook-in connectors (7) which are attached to vertical rails (8, 9), **characterised in that** a housing (2) is arranged on the chassis (1), which comprises a rear wall (3) as well as two side walls (4) and which is closed on its front side (5) by a single-wing or double-wing door, wherein the vertical rails (8, 9) are attached in the interior space of the housing and extend in parallel to the side walls (4) of the housing (2),
wherein the hook-in connectors (7), at one end, can be hooked into slots (10) in a vertical rail (8) arranged on the front side (5) of the housing and, at the other end, can be hooked into slots (10) of a vertical rail (9) adjacent to the housing rear wall (4), wherein the slots (10) of the front-side vertical rail (8) and the slots (10) of the vertical rail (9) adjacent to the housing rear wall are arranged in hook-in surfaces (11, 12) of the vertical rails (8, 9) which are aligned with each other in orthogonal direction,
**in that** the side walls (4) of the housing (2) on the front side (5) comprise a frame profile (14) U-shaped in cross-section,
**in that** the hook-in surface (11) of the front-side vertical rails (8) extends in parallel to the side wall (4) of the housing (2) and is formed onto the frame profile (14) in one piece or the front-side vertical rail (8) is configured as an angle profile and attached to the frame profile (14) and
**in that** the hook-in surface (11) of the rear-side vertical rails (9) adjacent to the rear wall (4) of the housing (2) is arranged at a distance in front of the rear wall (4).

2. The trolley according to claim 1, **characterised in that** the slots (10) of the vertical rails (8, 9) are vertically aligned.

3. The trolley according to claim 1 or 2, **characterised in that** the rear-side vertical rail (9) consists of an angle profile, which is attached to the side wall (4) of to a bar (13) bridging the housing corner.

4. The trolley according to one of claims 1 to 3, **characterised in that** the hook-in connectors (7) are manufactured as punched and bent components from sheet metal and at their ends comprise connecting lugs (15, 16) which can be fitted without a tool into the slots (10) of the vertical rails (8, 9).

5. The trolley according to claim 4, **characterised in that** the hook-in connectors (7) comprise a connecting lug (15), which is angled off at one end in an L-shaped manner and provided with an insertion slot, wherein the connecting lug (15) can be fitted onto the lower rim of the slots (10) in the vertical rails (8).

6. The trolley according to claim 5, **characterised in that** the hook-in connectors (7) comprise a connecting lug (16) at their other end, which extends in parallel to the side wall (4) of the housing (2) and comprises a width adapted to the length of the slots (10).

7. The trolley according to claim 5, **characterised in that** the hook-in connectors (7) comprise two connecting lugs (16, 16') at their other end, which extend in parallel to the side wall (4) and can be plugged into spaced-apart slots (10) of the vertical rail (9) .

8. The trolley according to claim 5, **characterised in that** one of the connecting lugs (16) has a width adapted to the length of the slots (10) and the other connecting lug (16') is narrower in shape.

9. The trolley according to one of claims 4 to 8, **characterised in that** the sheet thickness of the connecting lugs (15, 16) is chosen to match the gap width of the slots (10) provided in the vertical rails (8, 9) such that the connecting lugs (15, 16) are arranged in the slots (10) with a lateral play of less than 0.5 mm.

10. The trolley according to one of claims 1 to 9, **characterised in that** the slots (10) are arranged in the vertical rails (8, 9) with a uniform spacing chosen between 50mm and 70mm.

11. The trolley according to one of claims 1 to 10, **characterised in that** the hook-in connectors (7) comprise a central holding portion (17) set back in direction of the side wall (4) with a support surface for the support (6) and end portions (18) arranged in parallel to and offset from the holding portion.

12. The trolley according to one of claims 1 to 11, **characterised in that** the hook-in connectors (7) comprise a chamfer (19) at their lower rim for improving form stability.

13. The trolley according to one of claims 1 to 12, **characterised in that** the support (6) may be configured as a wire grid, a planar surface, a bowl or a basket and may be suspended from the hook-in connectors (7) or resting on the protruding surfaces of the hook-in connectors.

14. The trolley according to one of claims 1 to 13, **characterised in that** a glide rail (20) is attached to the hook-in connectors (7) and **in that** the support (6') comprises side parts (21) which are slidingy guided in the glide rail (20) between stop elements.

15. The trolley according to one of claims 1 to 14, **characterised in that** the glide rails (20) consist of plastic bars and **in that** profiles (23) are connected or moulded onto the side parts (21), which encompass the glide rails (20) in a U-shaped manner.

16. The trolley according to claim 15, **characterised in that** pegs (24) are attached to the underside of the glide rails (20), which limit the sliding path of the support (6').

17. The trolley according to one of claims 14 to 16, **characterised in that** the support (6') comprises a frame (22) formed from the side parts (21) and the cross members.

## Revendications

1. Chariot de transport, en particulier pour des produits stériles, avec un train de roulement (1) et au moins une tablette (6) réglable en hauteur, dans lequel la tablette (6) est en appui sur des traverses de suspension (7) fixées à des rails verticaux (8, 9),
**caractérisé en ce**
**qu'**un carénage (2) est disposé sur le train de roulement (1), lequel présente une paroi arrière (3) ainsi que deux parois latérales (4) et est fermé sur son côté frontal (5) par une porte à un ou deux battants, dans lequel les rails verticaux (8, 9) sont fixés dans l'espace intérieur du carénage et s'étendent parallèlement aux parois latérales (4) du carénage (2),
dans lequel les traverses de suspension (7) peuvent être suspendues à une de leurs extrémités dans des fentes (10) d'un rail vertical (8) disposé sur le côté frontal (5) du carénage et à leur autre extrémité, dans des fentes (10) d'un rail vertical (9) adjacent à la paroi arrière de carénage (4), dans lequel les fentes (10) du rail vertical (8) du côté frontal et les fentes (10) du rail vertical (9) adjacent à la paroi arrière de carénage sont disposées dans des surfaces de suspension (11, 12) des rails verticaux (8, 9), lesquelles sont orientées orthogonalement l'une par rapport à l'autre,
**que** les parois latérales (4) du carénage (2) présentent du côté frontal (5) un profilé d'encadrement (14) en forme de U en section transversale,
**que** la surface de suspension (11) des rails verticaux (8) du côté frontal s'étend parallèlement à la paroi latérale (4) du carénage (2) et est formée d'une seule pièce sur le profilé d'encadrement (14) ou que le rail vertical (8) du côté frontal est réalisé en tant que profilé angulaire et est fixé au profilé d'encadrement (14) et
**que** la surface de suspension (11) des rails verticaux (9) du côté arrière adjacents à la paroi arrière (4) du carénage (2) est disposée avec un espacement devant la paroi arrière (4).

2. Chariot de transport selon la revendication 1, **caractérisé en ce que** les fentes (10) des rails verticaux (8, 9) sont orientées verticalement.

3. Chariot de transport selon la revendication 1 ou 2, **caractérisé en ce que** le rail vertical (9) du côté arrière se compose d'un profilé angulaire qui est fixé à la paroi latérale (4) ou à un tasseau (13) enjambant l'encoignure du carénage.

4. Chariot de transport selon l'une des revendications 1 à 6, **caractérisé en ce que** les traverses de suspension (7) sont fabriquées en tôle en tant que pièces d'emboutissage et de cintrage et présentent des pattes de raccordement (15, 16) à leurs extrémités, lesquelles peuvent être insérées sans outil dans les fentes (10) des rails verticaux (8, 9).

5. Chariot de transport selon la revendication 4, **caractérisé en ce que** les traverses de suspension (7) présentent, à une extrémité, une patte de raccordement (15) coudée en forme de L et dotée d'une fente d'insertion, laquelle peut être fixée sur la bordure inférieure des fentes (10) dans les rails verticaux (8) .

6. Chariot de transport selon la revendication 5, **caractérisé en ce que** les traverses de suspension (7) présentent, à leur autre extrémité, une patte de raccordement (16) qui s'étend parallèlement à la paroi latérale (4) du carénage (2) et présente une largeur adaptée à la longueur des fentes (10).

7. Chariot de transport selon la revendication 5, **caractérisé en ce que** les traverses de suspension (7) présentent, à leur autre extrémité, deux pattes de raccordement (16, 16') qui s'étendent parallèlement à la paroi latérale (4) et peuvent être insérées dans des fentes (10) espacées entre elles du rail vertical (9) .

8. Chariot de transport selon la revendication 5, **caractérisé en ce que** l'une des deux pattes de raccordement (16) présente une largeur adaptée à la longueur des fentes (10) et l'autre patte de raccordement (16') est réalisée de manière plus étroite.

9. Chariot de transport selon l'une des revendications 4 à 8, **caractérisé en ce que** l'épaisseur de tôle des pattes de raccordement (15, 16) est adaptée à la largeur de fente des fentes (10) prévues dans les rails verticaux (8, 9) de manière à ce que les pattes de raccordement (15, 16) soient disposées dans les fentes (10) avec un jeu latéral de moins de 0,5 mm.

10. Chariot de transport selon l'une des revendications 1 à 9, **caractérisé en ce que** les fentes (10) sont disposées avec une dimension modulaire homogène sélectionnée entre 50 mm et 70 mm dans les rails verticaux (8, 9).

11. Chariot de transport selon l'une des revendications 1 à 10, **caractérisé en ce que** les traverses de suspension (7) présentent une partie de retenue (17) médiane en retrait en direction de la paroi latérale (4) avec une surface de support pour la tablette (6) et des parties d'extrémité (18) disposées de manière décalée parallèlement par rapport à la partie de retenue.

12. Chariot de transport selon l'une des revendications 1 à 11, **caractérisé en ce que** les traverses de suspension (7) présentent, sur leur bord inférieur, une partie pliée (19) pour l'amélioration de la stabilité de forme.

13. Chariot de transport selon l'une des revendications 1 à 12, **caractérisé en ce que** la tablette (6) est réalisée en tant que grille en fils d'acier, surface plane, bac ou corbeille et est suspendue aux traverses de suspension (7) ou repose sur des surfaces saillantes des traverses de suspension.

14. Chariot de transport selon l'une des revendications 1 à 13, **caractérisé en ce qu'**une glissière (20) est fixée aux traverses de suspension (7) et que la tablette (6') présente des parties latérales (21) qui sont guidées de manière coulissante au niveau des glissières (20) entre des éléments de butée.

15. Chariot de transport selon la revendication 14, **caractérisé en ce que** les glissières (20) se composent de tasseaux en plastique et que des profilés (23) sont en jonction avec les parties latérales (21), ou formés sur celles-ci, lesquels entourent les glissières (20) en forme d'un U.

16. Chariot de transport selon la revendication 15, **caractérisé en ce que** des tétons (24) sont fixés sur le côté inférieur des glissières (20), lesquels limitent le trajet de coulissement de la tablette (6').

17. Chariot de transport selon l'une des revendications 14 à 16, **caractérisé en ce que** la tablette (6') présente un cadre (22) formé par les parties latérales (21) et des supports transversaux.
